# EUROPEAN PATENT APPLICATION

(11) **EP 2 573 565 A1**
(43) Date of publication of application: **27.03.2013**
(21) Application number: 11007771.6
(22) Date of filing: 23.09.2011
(51) Int. Cl.: G01N 33/573

(54) **Immune detection method for common epitopes of two or more analytes in samples of complex compositions, device, and kit for enabling said immune detection method**

(71) Applicant: Kresbach, Gerhard Matthias, 79219 Staufen (DE); Ehrat, Markus, 4312 Magden (CH)
(72) Inventor: Ehrat, Markus, 4312 Magden (CH); Kresbach, Gerhard Matthias, 79219 Staufen (DE)

(57) **Abstract**

The invention relates to a method for detecting in an assay in a test sample one or more analytes, these analytes having at least one common epitope and one non-common linear or structural epitope. The method comprises the steps of providing a set of primary capture elements immobilized on a support, capable to bind to one or multiple non-common linear or structural epitopes of the one or more analytes in the test sample, and incubating the test sample with the immobilized primary capture elements for allowing binding of the one or more analytes to be detected to the immobilized primary capture elements.

The method according to the invention is characterized by the further steps of providing a set of one or more secondary capture elements capable to bind to common epitopes of the one or more analytes to be detected in the test sample and incubating said secondary capture elements with the test sample and the immobilized primary capture elements. Analytes exhibiting common epitopes and having been contained in the test sample and bound to the immobilized primary capture elements after incubation with said secondary capture elements are detected.

The invention also encompasses a device comprising a lateral flow device and a kit designed for allowing to perform the inventive method.

## Description

The present invention relates to a method for detecting in a test sample one or more analytes, these analytes having at least one common epitope and one non-common linear or structural epitope. The method comprises the steps of providing a set of primary capture elements immobilized on a support, capable to bind to one or multiple non-common linear or structural epitopes of the one or more analytes in the test sample, and incubating the test sample with the immobilized primary capture elements for allowing binding of the one or more analytes to be detected to the immobilized primary capture elements.

The present invention also encompasses a device designed for allowing to perform the inventive method, the flow device being designed to enable an immobilization of primary capture elements, and optionally of two or more analytes having at least one common epitope to be detected, on a support implemented in said flow device.

Furthermore, the invention encompasses a kit enabling performing the inventive method.

### BACKGROUND OF THE INVENTION

### Definitions

**Analyte** as used herein comprises the classes of proteins, peptides, carbohydrates, RNA, DNA, oligonucleotides, lipids, natural or synthetic organic molecules and polymers, complexes, conjugates which can have the cellular structure or functions such as hormones, signaling molecules, enzymes, antibodies, membrane receptors, and structural proteins. Analytes might be of synthetic origin or might derive - but not limited - from natural sources like blood, plasma, serum, cytosol, body fluids, tissue extracts, cell lysates, cultivation media and cultivation broths or might be chemical or biological modifications of material derived from natural sources.

**Capture element** as used herein comprises monoclonal and polyclonal antibodies, antibody fragments, synthetic antibodies, other functional or structural proteins, aptamers, oligonucleotides, DNA, lectins, natural and synthetic organic molecules, biotin-streptavidin, biotin-avidin, protein G, and protein A. Capture element can carry one or more detection labels.

**Detection label** as used herein comprises enzymes as applied in enzyme linked immunosorbent assays (ELISA), luminescent molecules and particles, chemiluminescence molecules and particles, vesicles or polymerosomes loaded with luminescence dyes, quantum dots and other nanoparticles, light scattering labels, surface enhanced raman scattering (SERS) labels, fluorescence resonance energy transfer (FRET) labels, labels that become a luminescence label upon cleavage, mass labels, radio and isotope labels. These detection labels can be directly linked to a capture element or indirectly linked to a capture element via a linker molecule, or via a high affinity complex such as streptavidin-biotin, avidin-biotin, digoxin-anti digoxin antibody or an anti species antibody or antibody fragment.

**Epitope** as used herein comprises the part of an analyte that binds to a particular capture element. Protein and peptide based epitopes may be either linear or structural. A structural epitope is typically composed of discontinuous sections of an antigen's amino acid sequence. These structural epitopes interact with the analyte(s) based on the 3-D surface features and shape or tertiary structure of the antigen. Linear epitopes interact with the analyte based on their primary structure. Amino acids that make up a linear epitope are a continuous sequence of amino acids of the analyte/antigen.

**Common epitope** as used herein comprises linear or structural epitopes that are present in two or more analytes and bind to the same capture element.

**Immuno assay** as used herein comprises a biochemical test that measures, typically, the presence or concentration(s) of one or more analytes that may be contained in a complex mixture of substances by using specific antigen - antibody interactions. A molecule that specifically binds to an antibody is called an antigen. Immuno assays can be carried out for either member of an antigen/antibody pair. For antigens, an antibody that specifically binds to that antigen can frequently be prepared for use as an analytical reagent. When the analyte is a specific antibody its antigen can be used as the analytical reagent. In addition to the need for specificity, a binding partner must be selected that has a sufficiently high affinity for the analyte to permit an accurate measurement. The affinity requirements depend on the particular assay format that is used.

**Immunohistochemistry** as used herein comprises a process of detecting antigens (e.g. proteins) in fixed or floating thin slices of a tissue by exploiting the principle of antibodies binding specifically to antigens present in these biological samples. Antibody interaction with the antigen of interest allows the determination of its local position in the tissue. Detection of an antibody-antigen interaction can be accomplished in a number of ways. In the most common instance, the antibody used is linked to a detection label.

**Multiplexed immuno assay** as used herein comprises solid or bead-based ("liquid") microarrays. Solid based microarrays comprise single sandwich, competitive or reverse assays in tiny spots localized statistically or geometrically at specific and known positions on an essentially planar surface. Each spot contributes to the information on a particular analyte.

**Liquid** microarrays comprise multiple sandwich or competitive assay formats, typically provided on beads, that can be identified typically by means of a color detection in the visible or infrared range. Each bead with a specific color contributes to the information on a particular analyte.

**Reverse array** as used herein means the variant of a multiplexed immuno assay where the complex sample, the content of which is provided either denatured or in its native conformation, is immobilized directly onto a solid support. Analytes of interest are specifically detected by the application of a capture element.

**Support** as used herein comprises essentially planar porous or non-porous solid surfaces, gels and gels on essentially planar surfaces, glass, polymer or magnetic beads which can be porous or non porous, membranes which can be porous or non porous, wells of micro and nano titer plates, and micro channels. Supports can be chemically, biochemically or physicochemically activated to improve the immobilization efficiency of analytes or capture elements.

**Western blot** as used herein comprises a gel based analytical technique used to detect specific proteins in a complex sample mixture. In a first step the proteins are separated either under native or denaturing conditions by gel electrophoresis according to their size, charge or other properties. In a second step the proteins are transferred from the separation gel to a detection membrane (typically nitrocellulose, polyamide, polyester, or polyvinylidene fluoride). Thus denaturing reagents can be removed and the separated proteins further analyzed using antibodies specific to the target protein in their native or denatured conformation.

### BACKGROUND OF THE INVENTION

### Immuno assay formats

For many fields of application, a multiplicity of biologically relevant analytes needs to be determined in a complex sample, for example, in diagnostic methods for determining an individual's state of health or in pharmaceutical research and development to find new drug targets and to measure the effects of the administration of biologically active compounds to an organism and investigating its complex functional mode.

The principle of immuno recognition offers the opportunity to quantitatively detect compounds utilizing so-called "capture elements" with high affinity and specificity to the analytes. Immuno recognition is particularly beneficial for the fast and economic detection of analytes in samples of complex composition and found widespread applications in medical diagnostics, in environmental sample analysis, in clinical diagnostics, as well as in several research and development areas. Several configurations of immune recognition methods exist, these methods comprising use of one or more capture elements:

Predominantly, a so-called "sandwich assay" format is used, in which a primary capture element, such as an antibody that is selected based on its specificity and binding affinity to a target molecule (antigen) as an analyte to be detected in a sample, is immobilized on a surface. A complex formed between one epitope of the antigen and the primary capture element is then exposed to a secondary capture element (tracer), e.g. a secondary antibody, that is able to interact with and bind to a second epitope of the antigen, thus forming a "sandwich complex". For certain detection principles such as energy transfer two tracer antibodies are applied for binding to two different epitopes of the analytes. To increase selectivity, a triple antibody ELISA assay utilizing fluorescence energy transfer was proposed in 2009 for diagnostic applications by Prometheus Laboratories San Diego, CA, USA (Profiling of Receptor Tyrosine Kinases (RTK) Activation in Circulating Tumor Cells (CTC) in Metastatic Tumors Using Proximity Mediated Microarray Immunoassays, Xinjun Liu et al.).

Sandwich immuno assays are among the most sensitive immune detection methods currently available, and quantification of the analytes in sandwich immuno assays is among the most accurate and precise known in immunodetection. Whereas sandwich immuno assays provide a high level of specificity and allow for reliable analyte quantification, they always require at least two highly specific antibodies recognizing different epitopes of the analyte. In case of intended detection and/or quantification of multiple analytes in a sample, for each analyte a pair of matching antibodies has to be developed and has to be implemented to an assay-related measurement kit. Whereas the sandwich assay format works well for single analyte assays, the potential for the detection of multiple analytes in one sandwich assay is limited since the development of sandwich immuno assays is tedious, time consuming, and not in all cases a suitable pair of primary and secondary antibodies can be discovered. Thus, proteomic investigations requiring detection and/or quantification of a large number of analytes in one sample, cannot be performed with a reasonable effort when using the sandwich assay format.

As one alternative to overcome the multiplexing issues of the sandwich immuno assays, the analytes are covalently bound to a detection label prior to the interaction with the primary antibodies immobilized on a support. Such type of products in a micro array format are available e.g. from Full Moon Biosystems (Sunnyvale, CA, USA) or from Sigma Aldrich (St. Louis, MO, USA), Panorama(TM) arrays. This allows the use of only one specific antibody per analyte. A signal indicative for binding of the analyte to the related and immobilized primary capture element is obtained from the label bound to the analyte. Whereas these capture assays require only one antibody specific for an epitope of the analyte, the modification of the analytes in the sample by the covalent binding to a detection label can change the epitope in a non-controlled manner and reduce or totally block the interaction with the specific antibody on the support. Since the sample composition is not well defined, the concentration of the analytes, varying from sample to sample, changes the stoichiometry of the reaction as well, thus making a reproducible and predictable labeling of the analytes difficult. The use of only one antibody specific for one analyte restricts the specificity of the assay compared to the sandwich format and requires a very thorough pretesting and selection of (primary) capture elements, particularly antibodies, regarding their analyte specificity and affinity.

Another alternative to overcome the multiplexing issues of sandwich immuno assays, comprise so-called "reverse assays". Reverse assays are typically performed in a multiplexed immuno assay format. Typically, in the course of reverse assays, one or more samples - containing mixtures of analyte molecules - such as cell-lysates, are adsorbed or covalently immobilized onto a solid support which may be chemically, biochemically, or physicochemically modified. Subsequently a specific (primary) antibody is applied to bind specifically to a selected epitope of one of the analytes. Typically, a secondary antibody (anti-species antibody), provided with a detection label and capable to bind to the primary antibody and possibly also to other antibodies of a class of antibodies containing the applied primary antibody, is applied for signal generation and detection.

Whereas reverse arrays require only one antibody specific for an epitope of the analyte, the accessible sample volume is limited, particularly for assays to be performed on supports provided as so-called "microarrays" on slides or comparable platforms, from some picoliters to a few microliters amounts (see WO 2005/095965) due to restrictions of sample spotting in an array format, making detection of low abundant proteins, e.g. inflammation markers such as interleukins, difficult or impossible.

The use of only one antibody specific for one analyte restricts the specificity of the assay compared to the sandwich format and requires a very thorough pretesting and selection of (primary) antibodies regarding their analyte specificity and affinity. The reverse array format, having the test samples immobilized on a support, requires to produce a large number of arrays and to analyze each array individually for a single analyte. The secondary antibody as used in reverse assays is intended for detection purposes only and does typically not bind to the analyte but to the primary antibody. Thus the assay specificity is dependent only on one antibody.

Another drawback of reverse assays (or microarrays prepared for reverse assays, in the context of this invention disclosure called "reverse arrays") is that an absolute quantification of the analyte is not easily possible. Several factors account for the difficulty to quantify: the adsorption of the analyte onto the surface depends on the matrix and buffer of the sample solution, on the variability of the surface properties, on washing and blocking protocols applied after the immobilization of the analyte on the surface. Reverse arrays have to be manufactured under well defined and controlled conditions and, but only after the samples becomes physically available, thus not allowing for an array preparation before availabilty of the actual samples to be analyzed. Thus, the complex manufacturing and handling procedure of reverse arrays has prevented their application in routine laboratories.

### Capture elements to interact with common epitopes of different analytes or antigens

In most cases the design, selection, and use of antibodies was targeted towards identification and monitoring individual antigens with high sensitivity and high precision for recognition of a single analyte epitope. In the last few years, however, the value of utilizing common epitopes of different antigens by means of group (or class) selective antibodies for preconcentration and preclassification prior to detailed analysis in the field of bioanalytics has been realized, and several methods for en the selection and production of antibodies recognizing common epitopes that are shared by a class of protein digests have been described (EP 1983002, US 6441140, US 7259022, US 2010/0074908, US 2009/0275013).

For unbiased discovery approaches in whole proteome analysis even highly sophisticated mass spectrometry approaches can hardly cope with the complexity of the samples containing the proteome of a cell. Thus, for approaches based on mass spectrometry, typically proteins are enzymatically digested and separated via chromatography prior to further analysis by mass spectrometry. Requirements to increase concentration of the analyte, demand for higher throughput, as well as the need for validation of the mass spectrometry results has lead to the introduction of affinity-based methods to capture so-called signature peptides derived from tryptic digests of biological samples in a single affinity step (US 7198896, US 7300753). According to a further approach (Molecular & Cellular Proteomics 10: 10.1074/mcp.M110.002857, 1-11, 2011), antibodies have been used and designed to recognize short C-terminal epitopes exhibiting sequences of three to four amino acid immobilized on a chromatographic support to isolate and enrich protein digests sharing the same terminal sequence prior to identification by mass spectrometry. In a similar fashion, antibodies have been used and designed to recognize C-terminal epitopes exhibiting sequences of four to six amino acids, immobilized on magnetic beads (Molecular & Cellular Proteomics mcp.M110.003932, 1-32, 2011).

Posttranslational modifications, such as phosphorylation of serine, threonine, and tyrosine and acetylation of lysine, play significant roles in cellular events, among others, control cell signaling pathways and regulations of major cellular functions. Typically, upon receiving a deactivating signal, the phosphorylated protein becomes dephosphorylated and suspends enzymatic activity; acetylation / deacetylation being predominantly involved in the regulation of transcription factors, effector proteins, molecular chaperones, and cytoskeletal proteins. The isolation of post translationally modified peptides and proteins and its subsequent analysis is therefore of high interest and several methods are known to specifically separate phosphorylated proteins based on their phospho Epitope (US 7300753).

A combination of 26 phospho specific antibodies was proposed in order to determine the global phosphorylation level of lysates derived from metastatic ovarian carcinoma tissue using reverse phase protein microarrays (Molecular & Cellular Proteomics 4, 346-355, 2005).

In US 2005/0153298 (Gembitsky et al.) a method is disclosed for selecting peptides and proteins to be captured by highly specific primary antibodies and certain specific phosphorylation motives measured by highly specific secondary antibodies that bind to posttranslationally modified amino acids independent of their position concerning the primary structure of the proteins. This allows to determine the amount of posttranslational modification, e.g. the percentage of phosphorylated tyrosine, for each of the proteins in a complex sample. Thereby, the procedure for selecting a highly specific antibody for, e.g., phosphorylated tyrosine containing epitopes independent of their position in the amino acid sequence and with as little cross reactivity as possible appears tedious. This makes the development and the selection of the secondary antibody, able to selectively recognize the phosphorylated tyrosine motive in many different proteins, extremely tedious.

In US 2010/0331199 (Stoll et al.) is disclosed the use of primary antibodies immobilized in a microarray format and targeted to recognize peptide epitopes that exhibit up to five, preferably two to three, recognition sites, preferably located at a terminal position of the peptide, to enrich peptides in a first step with low to moderate selectivity prior to subsequent further analysis. Upon exposure of the sample to the microarray exhibiting the primary capture elements (primary antibodies), in some embodiments disclosed in this patent application the captured peptides are detected using highly specific secondary capture elements.

### Correlation of common epitopes and biological function

The most widely used strategy to link sequence or structure of analyte epitopes to a function or homology-based function prediction, relies on the fundamental assumption that sequence or structural similarity implies functional similarity. This concept has been applied to the analysis and interpretaion of the function of kinases, members of the P450 superfamily of enzymes involved in first path metabolism, and G-protein coupled preceptors (GPCR). Correlation of linear or structural epitopes with function, however, so far could not be performed in a high-throughput fashion due to the limitations of mass-spectrometry-based approaches as described above.

**Kinases** are a family of enzymes whose catalytic domains are related in structure and sequence and further grouped into small families of related sequences i.e. TK, TKL, STE, CMGC etc. In general, members of the same kinase family have the same domain structure (Science 298, 1912-1934, 2002), in other words homology is inferred to all sequences in the same family (J. Mol. Biol. 320, 855-881, 2002). Whereas sequential information of kinases is readily available in databases such as SwissProt and other protein sequence collectiony, structural information is still limited. Monitoring of expression of proteins with common epitopes, be it linear or structural, in a simple and fast fashion, will allow quick survey of drug action, impact on cell signaling, and toxicological effects. In addition further grouping of kinase classes via homology could yield new drug targets.

### Kinase inhibitors

Many of the potent inhibitors discovered for a number of kinase targets have advanced to human testing, and a few have already been approved as drugs. Nearly all of the small molecule drug candidates in development block adenosine triphosphate (ATP) binding to the kinase as their primary mode of action. There is accumulating evidence that the activated (phosphorylated) kinases adopt very similar conformations whereas the non-phosphorylated kinases sample distinct conformational spaces within the open conformation (Analytical Biochemistry 373, 197-206, 2008).

Staurosporine is a prototypical ATP-competitive kinase inhibitor in that it binds to many kinases with high affinity, though with little selectivity. The main biological activity of staurosporine is the inhibition of protein kinases through the prevention of ATP binding to the kinase, achieved through the stronger affinity of staurosporine to the ATP-binding site on the kinase. The lack of specificity has limited its clinical use, but has made it a valuable research tool. Non selective compounds such as staurosporine can be used as capture element recognizing a common epitope, in this case the ATP binding site, of kinases.

### Objectives of the invention

It is a general objective of the invention to provide an alternative to well-established protein expression and activation profiling methods based on mass spectrometry, Western blots or reverse arrays, which is capable to measure high as well as low abundant proteins in high through-put and delivers precise quantitative results.

Particularly, it is an objective of the invention to provide an improved method for detecting one or more analytes in a test sample, implying reduced effort for detecting multiple analytes in a multiplexed format, thereby preserving the high selectivity, sensitivity and quantifiability as known from established sandwich immuno assay methods.

Still more particularly, it is a further objective of the invention to avoid the necessity of selecting two highly specific antibodies for one analyte and testing them before establishing an assay as known from the sandwich immuno assay methods of the state-of-the-art as discussed above.

Another objective of the invention is to provide a capability to detect multiple analytes in a sample using a single support provided as a microarray.

Furthermore, the invention shall allow to measure both denatured and not denatured analytes.

The invention shall also allow to combine the advantages of reverse arrays - namely ease of assay development once pretested antibodies are available - with the sensitivity, quantitative precision and reliability of sandwich immuno assays whilst maintaining all the general advantages of the array approach.

It is also an objective of the invention to provide a device for performing an assay method, according to the_invention, and a kit enabling performing the inventive method.

These objectives are realized by the characterizing features of the subject-matters of the independent claims. Further advantageous embodiments of the invention are specified in the dependent claims.

If not specified otherwise, all embodiments of the invention disclosed in the follwong can be combined with one another.

### Description of the invention

The invention provides a method for detecting in an assay in a test sample one or more analytes, these analytes having at least one common and one non-common linear or structural epitope. The method comprises the steps of a) providing a set of primary capture elements immobilized on a support, capable to bind to one or more non-common linear or structural epitopes of the one or more analytes in the test sample, and b) incubating the test sample with the immobilized primary capture elements for allowing binding of the one or more analytes to be detected to the immobilized primary capture elements. The method according to the invention is characterized by the further steps of c) providing a set of one or more secondary capture elements capable to bind to common epitopes of the one or more analytes to be detected in the test sample, and d) incubating said secondary capture elements with the test sample and the immobilized primary capture elements. Finally, according to the inventive method, analytes exhibiting common epitopes and having been contained in the test sample and bound to the immobilized primary capture elements after incubation with said secondary capture elements are detected.

In other words: The primary capture elements are selected based on their specificity and binding affinity to analytes of interest and are immobilized on the support. Optionally the support can be further coated with a blocking agent to minimize non-specific adsorption or a surface activating coating (the latter prior to the immobilization of the primary capture elements). The analytes in the test sample (sample solution) are added to the support containing the primary capture elements and incubated for a period of time for allowing the binding of analytes in the test sample to the primary capture elements.

A complex formed between a (non-common linear or structural) epitope of the analyte and the primary capture element is then exposed to a secondary capture element different from the primary capture element, does not interact with a potentially used blocking agent or the surface activating coating, if applied, and is able to interact with a second epitope common to analytes to be detected in the test sample, preferably common to two or more different analytes. The signal of the detection molecule is read by an appropriate equipment known to a person skilled in the art. Optionally known concentrations of molecules or compounds containing the common epitope of analytes to be detected in the test sample may be applied for calibration purposes to the surface supporting the primary capture elements.

Preferably, the primary capture elements comprise at least one of: monoclonal or polyclonal antibodies, antibody fragments, peptides, aptamers, functional proteins, DNA, oligonucleotides, or lectins.

In an advancement of the invention two or more (different) secondary capture elements capable to bind to two or more different common epitopes on one or more analytes are applied, and the identification of the binding of multiple (different) secondary capture elements to different common epitopes is achieved by using different detection labels. As an example:

Different kinases exhibit to a large extent common epitopes with three or four epitopes in length. Analysis of the sequences of human kinases has shown that nearly 50% of all kinases contain the 4 amino acid sequence HRDL, more than 20% the sequences VAIK or VAVK. More than 75% contain the 3 amino acid sequence RDL. AIK, AVK, ALK, and AMK combined are present in about 95% of all kinases. Combinations of antibodies recognizing such 3 and / or 4 amino acid sequence epitopes thus can be used for universal tracer assays for kinases.

In another variation of the invention two secondary capture elements against two different epitopes of an analyte are applied. At least one of the two capture elements being capable to bind to a common epitope of the analyte. Thereby prefarably, the two secondary capture elements may can carry suitable different detection labels to allow an energy transfer between these two detection labels.

Preferably, the analytes are of the classes of proteins and two or more of the analytes have a common, particularly linear, epitope accessible to a secondary capture element. Thereby, the preferred (identical or quasi-identical) sequence length of the common linear epitope is 2 to 20 (consecutive) amino acids, "quasi-identical" meaning in this context that certain amino acids may be different in the "quasi"-identical sequence as long as the differences still allow the binding of the secondary capture element to such common epitopes. For the interaction of secondary capture elements with linear common epitopes of analytes, these analytes are preferentially denatured before interaction with the secondary capture elements.

According to another preferred_variation of the invention, the analytes are of the classes of proteins, a common, particularly structural, epitope of at least two or more of these analytes being exposed to the secondary capture element in a native conformation, preferably such analytes being not denatured before interaction with the capture elements.

In preferred embodiments of the invention, the assays are performed in multiplexed arrangements, preferably such as microarrays, particularly the primary capture elements being provided on single wells of a micro or nano titerplate, on slides, membranes, or beads in the form of liquid arrays.

In preferred embodiments of the invention, the primary antibodies are selected according to their ability to bind to specific epitopes of cellular pathway proteins and are immobilized on a support. The primary antibodies may be arranged in sets reflecting certain signaling pathways, the primary antibodies may be specifically binding to posttranslationally activated proteins, to non-activated proteins and/or to epitopes on the protein that are not susceptible to posttranslational modifications. One or more secondary antibodies are selected according to their ability to bind to common epitopes of cellular pathway proteins. The secondary antibodies may be linked to one or more detection labels.

In a particularly preferred embodiment, the secondary antibodies are selected to identify certain classes of intra and extracellular proteins, such a class may comprise tyrosine protein kinases, mitogen activated protein kinases or serin/threonine protein kinases.

In an other preferred embodiment the secondary antibodies are selected to identify proteins involved in signaling pathways.

In other preferred embodiments of the invention, the assays are performed in lateral flow devices, specifically designed to enable the performance of the inventive method as described above and forming themselves another aspect of the invention. Typically the test sample is applied to the lateral flow device and encounters the secondary capture element, which may be linked to a label, binding to the common epitope(s) of one or more analytes, the complex being transported to the zone(s) with one or more immobilized primary capture elements, which may be spatially separated, binding to the specific epitope(s) of the analyte(s).

Alternatively the secondary capture elements binding to common epitope(s) of the analyte(s) are immobilized and secondary capture elements binding to common epitope(s) of the analyte(s) are forming the initial complex(es) that are transported to the detection zone(s). The primary capture elements may be linked to labels, which allow to discriminate between the analytes.

In general, secondary capture elements used to detect, preferably different, common epitopes of the analytes may be mixed together and supplied in form of a kind of "cocktail" of several individual secondary capture elements,

The secondary capture elements used to detect different common epitopes of the analytes may have a high cross reactivity.

The secondary capture elements used to detect common epitopes of analytes may be selected from the group of enzyme and drug target inhibitors. Such secondary capture elements for common epitopes may be selected from the group of inhibitor molecules binding to ATP binding sites of kinases like staurosporin. The capture elements selected typically exhibit dissociation constants in the range of low micro- to nanomolar.

For certain applications, secondary capture elements dedicated for binding to phosphorylated sites of an analyte, may be excluded.

The analytes to be decteted may be proteins, and their common epitopes may comprise "global" posttranslational modifications to be detected using secondary capture elements with high cross reactivity.

In a variation of the inventive method, a sum of phosphorylation sites irrespective of position and nature of amino acid in the peptide and protein sequence may be detected using such secondary capture elements, particularly antibodies, exhibiting high cross reactivity.

According to another variation of the invention, the primary capture elements comprise parts of or essentially the complete expressed proteome of an organism, immobilized on a support. Analytes to be detected and derived from natural or synthetic libraries of compounds are brought into contact with the primary capture elements and incubated for binding. Subsequently complexes formed between the primary capture elements and the analyte are brought into contact with secondary capture elements capable to bind to common epitopes of analytes, in order to recognize whole classes of analytes, e.g. kinases or phosphatases.

A further subject of the invention is a device comprising a lateral flow device dedicated for performing an inventive assay method according to any of the embodiments disclosed above. The flow device is designed to enable an immobilization of capture elements on a support implemented in the flow device, preferably allowing for immobilization of different primary capture elements binding to the specific epitope(s) of the analyte(s) in one or more spatially separated areas and alternatively of secondary capture elements, binding to common epitope(s) of the analyte(s). The secondary capture elements may optionally be used to concentrate a group of analytes of the test sample by trapping these analytes at certain defined zones.

A further subject of the invention is a kit enabling performing the inventive method according to any of the embodiments disclosed above. The kit comprises a support comprising immobilized (specific) primary capture elements and a reagent comprising the secondary capture elements to detect common epitopes of analytes contained in a test sample to be analyzed. The kit may comprise (different) secondary capture elements provided in a mixture like a cocktail, of several (different) individual capture elements to detect common epitopes of analytes. The secondary capture elements for detecting common epitopes of analytes may comprise enzyme and drug target inhibitors.

Core of the inventive idea is the identification of common epitopes of analytes and to detect such common epitopes using (secondary) capture elements, such as antibodies, capable to bind to said common epitopes of the analyte.

Accordingly, subject of the invention is also the application of this inventive idea to assay formats where the analyte itself is immobilized on a support or embedded in a matrix like a tissue slice, without using primary capture elements in the sense as above, namely as highly specific as possible to recognize an epitope on only one analyte, but using (secondary) capture elements capable to recognize and bind to common epitopes of multiple analytes.

Subject of such a further application of the general inventive idea is a method for detecting in a test sample one or more analytes having at least one common epitope, wherein the test sample is immobilized on a support. The method is characterized by the further steps of providing a set of one or more secondary capture elements, particularly antibodies, capable to bind to common epitopes of the one or more analytes to be detected in the immobilized test sample, and incubating said secondary capture elements with the immobilized test sample. Finally, according to the inventive method, analytes exhibiting common epitopes and having been contained in the test sample are detected after incubation with said secondary capture elements. In other words and more detail: The samples potentially containing analytes to be detected are preferably derived from biological materials, like cultures, primary cells, tissues, cytosol, blood, plasma, serum, cerebrospinal fluid, and are typically immobilized on a support in a dot blot or reverse array format. The analytes in the sample solution may optionally be denatured before the immobilization on the support. Optionally, after immobilization of the test sample, the support may be further coated with a blocking agent, as known to a person skilled in the art, to minimize non-specific adsorption. One or more secondary capture elements, particularly antibodies, capable to recognize one or more common epitopes of analytes to be detected, are added and incubated for binding to common epitopes. The identification of multiple (different) secondary capture elements, particularly antibodies, dedicated to bind to different common epitopes, is achieved by using different detection labels and appropriate related detection equipment. Preferably, thereby the number of applied (different) secondary capture elements is lower than the number of analytes to be detected. Optionally molecules containing the common epitopes to be detected are immobilized, prior to the application of the secondary capture elements, on the support from solutions of known concentrations to calibrate the measurements.

Particularly based on this embodiment of the inventive method, comparisons of analyte concentrations and amounts may be performed between samples derived from treated vs. non-treated cells and tissues, or between healthy and diseased tissues. Subject of a further application of the general inventive idea, particularly for immuno histochemistry assays, is a method for detection in a test sample one or more analytes having at least one common epitope, wherein the analytes are embedded in a matrix, preferably provided as a tissue slice. The method is characterized by the further steps of providing a set of one or more (secondary) capture elements, particularly antibodies, capable to bind to common epitopes of the one or more analytes to be detected and embedded in the support, and incubating said secondary capture elements with the support potentially containg analytes to be detected. Finally, according to the inventive method, analytes exhibiting common epitopes and_having been contained in the support, particularly a tissue slice, are detected after incubation with said (secondary) capture elements.

In other words and more detail: Analytes to be detected are contained in tissue slices prepared with a typical thickness between 4 to 40 µm and, if required, as in the case of formalin fixed paraffin embedded (FFPE) tissues, treated to make the epitope available for antibody binding as known to a person skilled in the art. Additionally, depending on the tissue type and the method of analyte, particularly antigen, detection, endogenous biotin or enzymes may need to be blocked or quenched, respectively, prior to antibody staining. Optionally, to reduce nonspecific binding, the tissue slices are incubated with a buffer that blocks the reactive sites but not the common epitopes. One or more antibodies capable to recognize one or more common epitopes of analytes to be detected are added and incubated for binding to common epitopes of analytes to be detected. The identification of multiple antibodies binding to different common epitopes is achieved by using different detection labels and appropriate related detection equipment as known to a person skilled in the art. Optionally molecules containing the common epitopes to be detected are immobilized, prior to the application of the secondary capture elements, on the support from solutions of known concentrations to calibrate the measurements.

According to another variation of the inventive method, the analytes are separated by gel electrophoresis and subsequently transferred to a membrane according to a Western blot approach. Optionally, in order to reduce nonspecific binding, the membrane is incubated with a buffer solution dedicated to block reactive sites but not the common epitopes of analytes to be detected. These Western Blots are incubated with one or more secondary capture elements, particularly antibodies, capable to bind to common epitopes of one or more analytes on the membrane. Optionally, molecules containing predefined common epitopes are added to the gel electrophoresis for reference and calibration purposes.

### Advantages provided by the invention

The present invention shall, in particular, contribute to improve and expedite the understanding of effects of external stimuli such as pharmaceutical compounds on cell and tissue activities.

The high selectivity achieved by the architecture of assays according to the present invention is due to the capture of analytes of interest by highly specific anti- analyte primary capture elements followed by the application of a secondary capture element with high cross reactivity binding to common epitopes of one or more analytes to be detected.

The present invention combines the high selectivity introduced, particularly by using highly specifically binding primary capture elements as known from established sandwich assay method and use of secondary capture elements that show a cross reactivity as high as possible to analytes of interest. The present invention therefore has five particular benefits compared to the method disclosed in US 2005/0153298 (Gembitsky et al.):
- The present invention uses the primary capture elements capable to selectively bind to phosphorylated as well as non phosphorylated sites of a selected protein, the primary capture element even being able to specifically bind to certain sequence specific sites which undergo posttranslation modifications.
- The present invention applies the secondary capture element mainly for detection purposes; therefore the secondary capture element should bind to as many of the sample proteins of interest as possible. A high cross reactivity is therefore a beneficial aspect of the secondary capture element as used in the present invention, since the selection criteria are less stringent.

- The present invention allows to fully denature the proteins prior to the execution of the assays and is not relying on structural epitopes only.
- The present invention allows to detect and quantify sequence specific posttranslational modification on only one array.
- The secondary capture elements may be mixed together in a detection cocktail, thus allowing to widen the scope of the detection on one array.

The present invention overcomes the disadvantages of reverse arrays, as disclosed above, by applying a primary single analyte specific capture element, contacting with a sample potentially containing analytes to be detected, followed by addition of a secondary capture element, particularly an antibody, specific for a common epitope of a class of analytes. The present invention has the advantages of high multiplexing capability of reverse arrays combined with the sensitivity to measure low abundance analytes, the selectivity and the quantitative precision and reliability of sandwich immuno assays.

The present invention allows to multiplex sandwich like immuno assays to a much higher degree, since the second capture element, particularly an antibody, is designed to bind to a common epitope of several analytes, and thus a "universal tracer assay" is designed to quantitatively identify groups of proteins with identical or quasi identical (common) structural or linear epitopes by two or more antibodies, one of them being identical for detection of two or more analytes. Problems related to different cross reactivities and binding kinetics of multiple secondary antibodies to different analytes in a multiplexed assay format are eliminated as well.

In contrast to US 2010/0331199 (Stoll et al.) the present invention uses highly selective primary capture elements to specifically capture analytes to be detected in a test sample and uses secondary capture elements capable to detect common epitopes for a general "broad" detection, not further restricted by specific selectivity of the applied secondary capture element, of the analytes specifically bound to the immobilized primary capture elemets.

In contrast to Stoll et al., according to the invention, neither primary nor secondary capture elements are to be restricted to the short length as described as described in US 2010/0331199, but, according to the invention, they are designed to be capable to bind to structural as well as linear epitopes of various lengths of peptides and proteins in a native as well as in a denatured state. Whereas in Stoll et al. a secondary highly specific capture element is used to identify individual analytes of interest, in the present invention the secondary capture elements are selected and used to identify and to be capable to detect multiple (different) analytes that exhibit common epitopes.

### Short description of the figures:

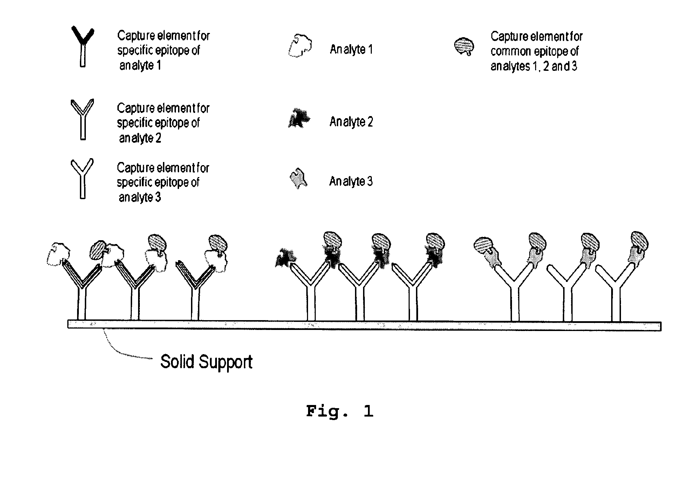

Fig. 1 shows an exemplary embodiment of the inventive immuno assay method.

### EXAMPLES

The invention is further explained in the following selected examples, without limiting the scope of the invention.

### Exemplary embodiment 1

Cultured HEK293 cells (ATCC, Manassas, VA, USA) were treated for 6 hrs either with 20 *µ*M GSK3 inhibitor SB216763 (Sigma-Aldrich) or with DMSO as a solvent control. After treatment, cells were washed twice with PBS buffer pH 7 and harvested. Cells are immediately lysed. Briefly, the cells were incubated for 10 minutes on ice in a denaturing lysis buffer (9.5M Urea, 2% CHAPS, 0.8% Pharmalyte, 1% DTT). Lysates were subsequently centrifuged at 15 000 rcf for 10 min at 4° C to remove any insoluble cell debris and the supernatants were retained. Acetone precipitation was performed by adding four volumes of ice cold acetone. After incubating at minus 20° C for 3 h, the samples were centrifuged at 15 000 rcf for 15 min at 4 C. The resulting pellets were resuspended in 100 *µ*L of PBS pH 7 and used for further analysis.

A kinase antibody array, containing specific antibodies against 276 human kinases (Kinase Antibody Array (Cat. Nr. AVK276) from Full Moon BioSystems (Sunnyvale, CA, USA) was used to determine the expression of kinases in cultured HEK293 cells:

The lysate solution, prepared as described above, was added to one or more microarrays with the immobilized antibodies and incubated over night at room temperature. The sample solution was then removed and the microarray surface rinsed 3 times with PBS buffer pH 7. An aqueous PBS buffered solution containing the secondary rabbit antibodies targeting the amino acid sequences VAIK, VAVK, VAVL, and YAMK were added and incubated for 8 hours at room temperature.

In a further step the secondary antibody solution is removed and the array is washed with 0.1 M PBS buffer pH 7. Subsequently an Alexa632-labeled anti rabbit antibody containing solution was added and incubated for 1 hour. Readout of the microarray was performed using a Gene Pix 4400A microarray scanner and Gene Pix Pro 3.0 Image analysis software (Molecular Devices, Sunnyvale, CA, USA).

### Exemplary embodiment 2

Cultured HEK293 cells (ATCC, Manassas, VA, USA) were treated for 6 hrs either with 20 µM GSK3 inhibitor SB216763 (Sigma-Aldrich) or with DMSO as a solvent control. After treatment, cells were washed twice with ice-cold PBS buffer pH 7 and resuspended in 250 *µ*l of ice-cold RIPA buffer (50 mM Tris buffer, pH 7.4, 1% IGEPAL, 0.25% sodium deoxycholate, 150 mM NaCl, 1 mM EDTA, 1 mM NaF, 1 mM PMSF, 1 mM Na₃VO_{4,} containing Complete (TM) protease inhibitor mixture). Cells were lysed for 15 min on a shaking platform at 4 °C, centrifuged at 14,000 g for 15 min at 4 °C, and the supernatant used for further analysis.

A protein microarray array was produced by spotting antibodies against 100 selected MAP Kinases (Cell Signaling Technologies, Danvers, MA, USA) dissolved in 0.1 M Phosphate buffer pH 8.5 at a concentration of 1 mg/ml onto a NEXTERION MPX-16 Slide H 75.6 mm x 25.0 mm glass slide (Schott, Jena, Germany) using the tip-based Dip Pen Nanolithography^{®} (DPN^{®}) technology NanoArrayer 3000 (NanoInk, Skokie, IL, USA). The chip surface was blocked with a bovine serum albumin according to standard procedures.

The lysate solution, prepared as described above, was added to one or more microarrays with the immobilized antibodies and incubated over night at room temperature. The sample solution was then removed and the microarray surface rinsed 3 times with 0.1 M PBS buffer pH 7. The secondary antibodies targeting the amino acid sequences VAIK, VAVK, VAVL, YAMK, and HRDL, and labeled with Alexa 632, were added and incubated for 8 hours at room temperature.

Readout of the microarray was performed using a Gene Pix 4400A microarray scanner and Gene Pix Pro 3.0 Image analysis software (Molecular Devices, Sunnyvale, CA, USA).

### Exemplary embodiment 3

Cultured HEK293 cells (ATCC, Manassas, VA) were treated for 6 hrs either with 20 *µ*M GSK3 inhibitor SB216763 (Sigma-Aldrich) or with DMSO as a solvent control. After treatment, cells were washed twice with ice-cold PBS buffer pH 7 and resuspended in 250 *µ*l of ice-cold RIPA buffer (50 mM Tris buffer, pH 7.4, 1% IGEPAL, 0.25% sodium deoxycholate, 150 mM NaCl, 1 mM EDTA, 1 mM NaF, 1 mM PMSF, 1 mM Na₃VO_{4,} containing Complete (TM) protease inhibitor mixture). Cells were lysed for 15 min on a shaking platform at 4 °C, centrifuged at 14,000 g for 15 min at 4 °C, and the supernatant used for further analysis.

A protein microarray array was produced by spotting antibodies against 100 selected MAP Kinases (Cell Signaling Technologies) dissolved in 150 mM Phosphate, pH 8.5, 0.01 % Tween^{®} at a concentration of 1 mg/ml onto a NEXTERION MPX-16 Slide H 75.6 mm x 25.0 mm glass slide (Schott, Jena, Germany) using a GeSiM NanoPlotter NP2.1 (GeSiM, Großerkmannsdorf, Germany) and spotting volumes of approximately 0.5 nl. The chip surface was quenched with 25 mM ethanolamine in 100 mM sodium borate buffer pH 8.5 and blocked with a bovine serum albumin solution.

The lysate solution prepared as described above was added to a microarray with the immobilized antibodies and incubated over night at room temperature. The sample solution was then removed and the microarray surface rinsed 3 times with PBS buffer pH 7. A mixture of secondary anti AIK, AVK, AVL, AMK, and HRD antibodies, labeled with Alexa 488, 532, 594, 632 and 647 respectively was added and incubated for 8 hours at room temperature.

Readout of the microarray was performed using a Gene Pix 4400A microarray scanner and Gene Pix Pro 3.0 Image analysis software (Molecular Devices, Sunnyvale, CA, USA).

### Detailed description of the figures

Fig. 1 shows surface immobilized primary capture elements, located in three spatially separated areas, binding to the specific epitope(s) of the three different analytes. The capture element for common epitope binds to the common epitope of the three analytes to form a sandwich "primary capture element - analyte - secondary capture element".

## Claims

1. A method for detecting, in an assay, in a test sample one or more analytes having at least one common and one non-common linear or structural epitope, the method comprising the steps of:
a. providing a set of primary capture elements immobilized on a support, capable to bind to one or more non-common linear or structural epitopes of the one or more analytes in the test sample; and
b. incubating the test sample with the immobilized primary capture elements for allowing binding of the one or more analytes to be detected to the immobilized primary capture elements,
**characterized by the further steps of**
c. providing a set of one or more secondary capture elements capable to bind to common epitopes of the one or more analytes to be detected in the test sample;
d. incubating said secondary capture elements with the test sample and the immobilized primary capture elements; and
e. detecting analytes exhibiting common epitopes and having been contained in the test sample and bound to the immobilized primary capture elements after incubation with said secondary capture elements.

2. A method as claimed in claim 1,
**characterized in that**
the assays are performed in multiplexed arrangements on beads or in an array format on slides, single wells of a micro or nano titerplate.

3. A method as claimed in any one of the preceding claims, **characterized in that**
- the primary antibodies are selected according to their ability to bind to specific epitopes of cellular pathway proteins and are immobilized on a support;
- one or more secondary antibodies as secondary capture elements are selected according to their ability to bind to common epitopes of cellular pathway proteins, The secondary antibodies being selected to identify certain classes of cellular proteins, comprising tyrosine protein kinases or mitogen activated protein kinases or serin/threonine protein kinases, or particular signaling pathway proteins.

4. A method as claimed in any one of the preceding claims, **characterized in that**
the secondary antibodies are selected to identify one or more of the common epitopes of kinases having the amino acid sequences VAIK, VAVK, VAL, YAMK, and HRDL or part of these.

5. A method as claimed in any one of the preceding claims, **characterized in that**
the analytes to be detected in the test sample are proteins, particularly the common epitopes comprising global posttranslational modifications.

6. A method as claimed in any one the preceding claims, **characterized in that**
the analytes to be detected are proteins and have a common linear epitope with a length of 2 to 20 consecutive amino acids.

7. A method of claim as claimed in any one the preceding claims, **characterized in that**
the common epitopes are presented in a native conformation for exposure to the secondary capture elements, which might not have consecutive amino acid sequences to form this common epitope.

8. A method as claimed in any one of the preceding claims, **characterized in that**
the assays are performed in lateral flow devices, designed to enable the performance of a method as claimed in any one of the preceding claims,
**characterized by the further steps of**
- applying the test sample to a lateral flow device for encountering the secondary capture elements which may be linked to a label,
- allowing for binding to common epitopes of one or more analytes, a formed complex between an analyte and a secondary capture element being transported to zones with one or more immobilized primary capture elements, which are preferably spatially separated,
- allowing for binding of specific epitopes of the at least one analyte to primary capture elements;
or
- in a first step immobilizing secondary capture elements capable for binding to one or more common epitopes of the at least one analyte,
- allowing for forming initial complexes between the analyte and the secondary capture elements followed by transporting them to one or more detection zones, wherein the primary capture elements may be linked to labels, which allow to discriminate between different analytes.

9. A method for detecting in a test sample one or more analytes having at least one common epitope, wherein the test sample is immobilized on a support or embedded in a matrix, particularly in a tissue slice,
the method comprising and being
**characterized by the steps of:**
a. providing a set of one or more capture elements, particularly antibodies, capable to bind to common epitopes of the one or more analytes to be detected in the test sample,
b. incubating said secondary capture elements with the test sample, and
c. detecting analytes exhibiting common epitopes and having been contained in the test sample after incubation with said secondary capture elements.

10. A kit enabling performing a method according as claimed in any one of claims 1 to 9, the kit comprising a support comprising immobilized primary capture elements and a reagent comprising secondary capture elements to detect common epitopes of analytes contained in a test sample to be analyzed.

11. A kit as claimed in claim 10, comprising a support with immobilized primary antibodies specifically binding to posttranslationally activated proteins, to non-activated proteins and/or to epitopes on a protein that are not susceptible to posttranslational modifications, and a reagent containing secondary antibodies as secondary capture elements against one or more common epitopes of cellular pathway proteins.

12. A kit as claimed in claim 10 or claim 11,
**characterized in that**
the secondary capture elements for detecting common epitopes of analytes comprise enzyme and/or drug target inhibitors.

13. A kit as claimed in any one of claims 10 to 12 comprising a support with immobilized primary antibodies as primary capture elements specifically binding to posttranslationally activated proteins, to non-activated proteins and/or to epitopes on the protein that are not susceptible to posttranslational modifications, and a reagent containing secondary antibodies as secondary capture elements against one or more common epitopes of cellular pathway proteins.

14. A kit as claimed in any one of claims 10 to 13, comprising secondary capture elements provided in a mixture of several individual different secondary capture elements that may or may not carry different labels to detect common epitopes of analytes.

15. A device comprising a lateral flow device enabling performing a method according as claimed in any one of claims 1 to 9, the flow device being designed to enable an immobilization of capture elements on a support implemented in the flow device, preferably allowing for immobilization of different primary capture elements binding to at least one specific epitope of at least one analyte in one or more spatially separated areas, and alternatively of secondary capture elements capable for binding to one or more common epitope(s) of the at least one analyte, particularly for concentrating a group of analytes of the test sample by trapping these analytes at certain defined zones.
